# EUROPEAN PATENT SPECIFICATION

(11) **EP 4 054 417 B1**
(45) Date of publication and mention of the grant of the patent: **17.01.2024**
(21) Application number: 20807911.1
(22) Date of filing: 05.11.2020
(51) Int. Cl.: A61B 5/15

(54) **BLOOD COLLECTION DEVICE AND METHOD FOR THE SELF-COLLECTION OF BLOOD BY A USER**
BLUTENTNAHMEVORRICHTUNG UND VERFAHREN ZUR SELBSTENTNAHME VON BLUT DURCH EINEN BENUTZER
DISPOSITIF DE COLLECTE DE SANG ET PROCÉDÉ D'AUTO-COLLECTE DE SANG PAR UN UTILISATEUR

(30) Priority: 07.11.2019 NL 2024178
(43) Date of publication of application: 14.09.2022
(73) Proprietor: Stichting Het Nederlands Kanker Instituut- Antoni van Leeuwenhoek Ziekenhuis, 1066 CX Amsterdam (NL)
(72) Inventor: VAN DEN BRINK, Nicas, 1066 CX AMSTERDAM (NL); VAN ROSSUM, Huub Harmen, 1066 CX AMSTERDAM (NL)
(74) Representative: Algemeen Octrooi- en Merkenbureau B.V.
(86) International application number: PCT/NL2020/050691
(87) International publication number: WO 2021/091380

(56) References cited:
- EP-A1- 0 173 811
- WO-A1-03/005909
- WO-A1-2018/039307
- US-A- 3 623 475

## Description

The present invention relates to a blood collection device.

Such devices are known in general and can typically be categorised into two categories. On the one hand, there are devices which allow the self-collection of a blood sample by a user, i.e. the collection of a blood sample by a user without any help of a second person such as a physician. These devices are however usually rather complex, and therefore relatively costly. Another disadvantage of such devices is that they are typically relatively large, which makes it impossible to send these devices to users via letterbox post. Instead, it has to be send as a package (which of course results in higher cost). US 2015/0351676 A1 e.g. describes such a device.

On the other hand there are devices which are smaller, but these typically cannot be used by a user for the self-collection of blood. That is, these types of devices require a second person, such as a physician, to successfully collect a blood sample. With such devices, typically three hands (and, thus, at least two persons) are needed. Firstly there is the hand comprising the finger of the user from which the blood sample is to be taken. Secondly, there is a hand which is needed to "push out" the blood from the finger of the user. And thirdly there is a hand which is needed to hold the blood collection device and collect the blood sample. The requirement of three hands makes it rather impossible for users to collect blood themselves with such devices. For example the web page https://www.lumc.nl/patientenzorg/praktisch/patientenfolders/bloe-prikken-voor-HbA1C-bepaling-instructie-voor-thuis (as accessed on the date of filing) shows such a device.

WO03/005909 A1 discloses a blood specimen sampling and insulated shipping kit that provides a user with all materials that are needed to initiate blood flow, direct a blood sample into a sample vial, insulate the vial from heat and cold, protect the vial from impact, and ship the blood sample containing vial to a clinical laboratory for testing.

US3,623,475 discloses a tube for use in the automatic collection of a digital blood sample which has its mouth fitted with a resilient detachable funnel member which can carry a capillary cannula and in which is held an absorbent anular plug through which pass and project two incisor lancets.

EP 0 173 811 A1 discloses a biological specimen collection device that comprises a specimen receptacle having a closed first and an open second end. A removeable conduit is provided for introducing a biological specimen into the receptacle and is connected to the second end of the receptacle. A base removably receives the first end of the receptacle and supports the receptacle in a substantially vertical position. A cap is removably connected to the base, this cap is adapted to provide a liquid seal between the cap and the open second end of the receptacle. The cap is maintained in a protective environment during introduction of the biological specimen into the receptacle. Access is permitted to the cap after the specimen has been collected for subsequently capping the open end of the receptacle with its contents.

WO 2018/03937 A1 discloses a collection device which directs a flow of blood into a container and provides a controlled blood flow path that ensures blood flow from a collection site to a collection container.

Hence, there is a need for a device which combines the good aspects of both these types of devices, i.e. there is a need for a device which allows a user to collect a blood sample himself (herself), and which is sufficiently compact to allow it to be send via lettermail post.

It is an object of the present invention to provide such a device.

Therefore, a blood collection device in accordance with claim 1 is provided.

The blood collection device according to the invention advantageously is a very simple device, having only a few components, and having low production cost. The device is very simple to assemble for a user, and may advantageously be shipped to said user via letterbox post, such that shipping costs are minimal. In use of the blood collection device advantageously a "third hand" for the collection of a blood sample is not needed so that the device may truly be used for the "self-collection" of a blood sample by the user himself (herself).

The device according to the invention comprises a base element, which base element is a separate part compared to the other components of the blood collection device in the sense that it can be de-assembled and assembled together with the other components of the blood collection device to form the blood collection device in an assembled state. With reference to the below text where this is explained in more detail, it is noted that the other components of the blood collection device may also be de-assembled with respect to each other, but this is not required. Some or all of the other components may be integrated with each other.

The base element is configured to be shipped to a user in a de-assembled state with respect to the other parts, such that a small and comprehensive package results in the de-assembled state of the blood collection device, a package which may be send through lettermail post (which according to Dutch regulations in force at the filing date of the present application means at least that the height of the package should be less than 32 mm). In other countries other requirements may however apply for lettermail post and the design of the blood collection device may easily be adapted to such requirements without departing from the inventive concept underlying the invention. Purely as an example and to the best knowledge of the applicant, the European standard for letter boxes, EN 13724:2002, as in force on the date of filing, mentions an aperture height of 30-35 mm; Canadian law mentions a height of 40 mm on the filing date; USA law mentions a height of 1.75 inch (about 44.5mm) on the filing date; and Australian law mentions a height of 30mm on the filing date.

The base element is further configured to be assembled with the other parts of the blood collection device by a user after said user has received the blood collection device via lettermail post and before using said blood collection device. As is explained in more detail below, the design of the blood collection device is advantageously very simple such that any user is able to assemble the blood collection device without difficulties and without the risk of a misassembly.

Alternatively phrased, the blood collection device can be de-assembled (it is however by no means required that the device has been assembled before being de-assembled), e.g. to ship it to a user in a de-assembled state, and the blood collection device can be assembled, e.g. to use it for collecting a blood sample in an assembled state.

It has been an important insight of the inventors that a base element having a width of more than 32 mm ensures a stable support surface when the blood collection device is used, and that the possibility to de-assemble it with respect to the other components of the blood collection device makes it possible to send the base element via lettermail post at the same time. In other words, by providing a blood collection device that can be de-assembled, the two functions of providing a stable support and being sufficiently compact to allow shipping via letterbox post can be fulfilled at the same time. Compared to the solutions of the prior art, the stable support by the base element ensures that no "third" hand of a user (or a second person) is needed to hold the blood collection device. Instead, the blood collection device is self-supporting. This makes the collection of blood with just two hands, i.e. by the user him/herself possible.

The base element should then have a height of less than 32 mm to allow it to be send via lettermail post in the de-assembled state. As the width is more than 32 mm, it is not possible to send the blood collection device via lettermail box in its assembled state and therefore, advantageously, it is possible to de-assemble the base element with respect to the other elements of the blood collection device.

In the assembled state of the blood collection device, the base element may form a stable foot which may be placed by a user on a support surface such as a desk, a table, or another relatively large, substantially horizontal surface. The collection container is placed inside the base element, in the receive opening thereof, and extends upwards with respect to the base element.

In use, a user of the blood collection device may typically assemble the blood collection device, prick his/her finger with a lancet, wait and/or work the finger until a drop of blood is visible, place said finger on the finger portion of the blood collection device, and wait until enough blood is drawn from the finger of the user into the collection container through the fluid channel. The upright positioning of the collection container ensures that blood drawn from the finger of the user is transported towards the collection container along with the force of gravity, such that actively pushing blood out of the finger of the user may be needed to a lesser degree or not at all, and hence allowing the "self-collection" of a blood sample.

For example, the user may prick the ring finger or the middle finger with the lancet, e.g. in a top section thereof.

Advantageously, the blood collection device does not comprise any moving parts, such that both assembly and manufacturing are fast and cheap.

The finger portion comprises a blood collection channel including a blood collection hole, a width of the blood collection channel decreasing in a direction from an upper surface of the finger portion to the collection container. At the bottom surface of the finger portion, the blood collection hole extends into a blood collection channel, the channel extending towards the collection container in the assembled state of the blood collection device. A cross-section of said channel is larger near the top thereof (i.e. near the finger portion) than near the bottom thereof (i.e. near the collection container). For example, the cross-sectional shape of the channel may be tapered or conical. It was surprisingly found by the applicant that such a shape allows any air present in the collection container after assembly to leave the collection container, allowing the blood to more easily flow through the blood collection hole (channel), the fluid channel, and into the collection container. An almost immediate flow is observed once the user places his/her (pricked) finger on the finger portion. The specified shape of the blood collection channel furthermore helps to efficiently direct blood towards and into the collection container. The specified shape also helps to direct blood towards and into the collection container in a clean way, i.e. with less sloppiness.

In other words, a blood collection channel having a width which decreases in a direction from an upper surface of the finger portion to the collection container provides an air outlet channel for any excess air in the collection container after assembly.

In an embodiment, the blood collection device further comprises a capillary tube that, in an assembled state, is arranged between the blood collection hole and the collection container, the capillary tube defining said fluid channel. When a capillary tube is provided, it becomes very easy to collect relatively small blood samples.

In an embodiment, the capillary tube extends into the collection container, the collection container having a larger width (diameter) than the capillary tube.

In an embodiment, an outer width of the capillary tube is larger than a width of the blood collection hole. After assembly, air may be present in the collection tube and/or the internal space of the finger portion. This air may create a relative overpressure which prevents blood deposited on the finger portion by the user to easily flow into the collection container. When the outer width of the capillary tube is larger than a width of the blood collection hole, this prevents the capillary tube to fill and obstruct the blood collection hole / blood collection channel. This in turn allows any air in the collection container / internal space of the blood collection device to leave through the blood collection hole, so that an overpressure in the collection container / internal space of the finger portion is reduced and a more easy flow of blood into the collection container may be obtained.

In other words, an outer width of the capillary tube being larger than a width of the blood collection hole provides an air outlet channel for excess air in the collection container after assembly.

In an embodiment a side wall of the finger portion comprises an air outlet hole. Said air outlet hole may act as an air vent to allow any air residing in the collection container and/or the internals of the blood collection device (that creates an overpressure) to escape therefrom, so that a more easy flow of blood from the finger portion into the collection container may be obtained. In other words, the air outlet hole provides an air outlet channel for excess air in the blood collection device after assembly. Advantageously, when said air outlet hole is provided in the side wall of the finger portion, it cannot be obstructed by blood and is always free.

In an embodiment the blood collection device further comprises an insert member configured to be inserted into the finger portion and for receiving the capillary tube, to fixate the capillary tube with respect to the finger portion. The insert member may be made of a different material than the finger portion. For example, the finger portion may be made form a relatively stiff material, and the insert member may e.g. be made from a relatively flexible material. This allows to pressure-fit the insert member with respect to the finger portion, and to pressure-fit the capillary tube with respect to the insert member, and thus to secure the capillary tube with respect to the finger portion. For example the insert member may be pressure-fit in a hollow side wall (or a hollow internal wall) of the finger portion. For example, the capillary tube may be pressure-fit in a capillary tube receiving channel of the insert member. The insert member may have recesses in its outer circumferential surface, which recesses may define air outlet channels for excess air in the collection container after assembly.

In this embodiment, preferably the capillary tube receiving channel of the insert member has a non-circular, e.g. square, cross-section, and the capillary tube has a circular cross-section. This allows on the one hand to pressure-fit the capillary tube in the insert member, e.g. when the outer diameter of the capillary tube is approximately the same as an inner width of the capillary tube receiving channel. This allows on the other hand that an outflow channel for any air in the collection container is provided between the outer side of the capillary tube and the capillary tube receiving channel. The open space between the outer side of the capillary tube and the capillary tube receiving channel defines an air outlet channel.

Alternatively, a capillary tube receiving channel of the insert member may have a circular cross-section while the capillary tube has a non-circular cross section, and wherein the sizes of the capillary tube and the capillary tube receiving channel are matched to each other to allow a press-fit of the capillary tube in the capillary tube receiving channel.

Preferably, when the blood collection device comprises an insert member, the finger portion, the insert member and the capillary tube are pre-assembled in the de-assembled state. Alternatively, when the blood collection device does not comprise an insert member, the finger portion and the capillary tube are pre-assembled in the de-assembled state. As explained in the above, it may be desirable that any air residing in the collection container after assembly can leave the collection container. Therefore, flow channels for excess air (i.e. air outlet channels) are preferably provided, where several different embodiments of such flow channels are described in the above. One or more of such flow channels for air may be present in the blood collection device. To ensure that the flow channels are not closed as a result of incorrect assembly, preferably the capillary tube is pre-assembled with respect to the finger portion in the de-assembled state. Accordingly, in the de-assembled state not all components of the blood collection device need to be separated from each other. Some of the components may be pre-assembled in the de-assembled state.

In an embodiment an upper surface of the finger portion has an elongate shape. Advantageously an elongate shape makes it easy and intuitive for a user to place his/her finger on the finger portion, as a finger also has an elongate shape. The finger portion may e.g. have an elliptical, rounded shape. The finger portion may e.g. have an upper surface which is slanted towards the blood collection hole / blood collection channel. This matches the natural shape of a finger, and transports any blood automatically towards the blood collection hole. The finger portion may have an upstanding wall portion. The finger portion may have a "lip" where a root of the finger is to be placed, whereas the blood collection hole is arranged at the opposite side of the lip. All these factors, individually and combined, may make it easy and intuitive for a user to place a finger of the user on the finger portion in the most optimal way, and to collect the blood sample in the most efficient way, offering a pleasant experience for the user. To provide a stable and secure structure for the placement of a finger, a length of the finger portion may be more than 32 mm, e.g. more than 35 mm, e.g. about 38 mm. In that case, the finger portion is preferably send in a de-assembled state with respect to the collection tube and preferably also with respect to the base element. Preferably a height of the finger portion is less than 32 mm, to allow it to be shipped via lettermail post in a de-assembled state.

Advantageously, the elongate shape of the finger portion, and the placement of the blood collection hole, ensures that a user intuitively knows how to place the (pricked) finger on the finger portion for the sampling of blood.

In an embodiment, a width of the base element is larger than 40 mm, preferably larger than 50 mm, such as larger than 55 mm. In general, a wider base element will result in a more stable blood collection device. Preferably, a circumferential shape of the base element is circular, but other shapes are well conceivable. Preferably the width of the base element exceeds the length of the finger portion.

In an embodiment, the base element comprises a receive pocket including the receive opening for receiving the collection container in the assembled state of the blood collection device. The receive pocket may e.g. be formed by an internal wall of the base element. A receive pocket ensures a stable and secure assembly of the collection container with respect to the base element, as the contact area between the base element and the collection container is increased compared to when e.g. only a receive opening is defined in the base element for receiving the collection container.

In an embodiment the collection container comprises at least one barline for indicating a fill level of the collection container. Preferably, the collection container is transparent. The barline may indicate the level till which the container is to be filled, e.g. 50 µl, 100 µl or 200 µl. For different tests, different amounts of blood may be desired. Therefore the collection container may comprises several such barlines, each indicating a different fill level. Instructions may then be provided along with the blood collection device regarding the amount of blood that is to be collected. To also see whether the amount of blood collected is above or at the required fill level, the collection container is preferable transparent, although other ways to indicate this are known to a person skilled in the art.

In an embodiment the base element and/or the finger portion are manufactured via an injection moulding process, the base element and the finger portion preferable being made from polypropylene. An injection moulding process typically requires relatively expensive moulds and thus a relatively large initial investment, but has a relatively low price per piece such that an injection moulding process may be cost-effective when a large amount of devices, e.g. more than 10,000, is produced.

In an alternative embodiment the base element and/or the finger portion are manufactured via an 3D printing process, preferably via a selective laser sintering process, the base element and the finger portion preferable being made from PA2200. A 3D printing process has a relatively low initial investment (only the 3D-printer, which is cheap compared to an injection mould), but has a relatively high price per piece such that a 3D printing process may only be cost-effective when a relatively low amount of blood collection devices is made, e.g. less than 1,000. For customization purposes, it may however be desired to have small batches such that 3D printing a desirable. This e.g. allows branding to be provided on the side of the blood collection device. A particular advantage of PA2200 is that it is a biocompatible plastic.

A second aspect of the present invention relates to a base element for a blood collection device, the base element having a width of more than 32 mm and including an upstanding wall which defines a receive opening for receiving a collection container or a blood collection device, the upstanding wall having a height of less than 32 mm,
wherein the base element is a separate part, configured to be shipped to a user in a de-assembled state with respect to the other parts of the blood collection device or with respect to the blood collection device, and
wherein the base element is configured to provide a stable support for the blood collection device in an assembled state.

Making use of the same inventive concept as described in relation to the first aspect of the invention, any known blood collection device may be provided together with a separate base element that is configured to provide a stable support. Any blood collection device can then be placed on a support and be used by a user for the self-collection of a blood sample without the need of a "third hand" or second person. The base element of this second aspect may be provided "in addition to" a blood collection device, or may be provided "as a part of" a blood collection device.

A third aspect of the present invention relates to a blood collection kit comprising the blood collection device as detailed in the above, a lancet, and a cap for sealing the collection container. Preferably the blood collection kit is packed in a sterilized bag. Optionally the blood collection kit also comprises a return envelope and/or an analysis tool for self-analysis of the collected blood sample.

A fourth aspect of the present invention relates to a method for collecting a blood sample, comprising the steps of:
- shipping a blood collection device as detailed in the above or a blood collection kit as detailed in the above to a user by letterbox post, a base element of the blood collection device being de-assembled with respect to the other parts of the blood collection device during shipment,
- assembling the blood collection device;
- pricking a finger of a patient to initiate a blood flow;
- placing the finger of the patient on the finger portion of the blood collection device;
- collecting the blood of the patient in the collection container;
- de-assembling the blood collection device;
- returning the collection container, inclusive of the blood collected therein;
wherein the steps of assembling the blood collection device, pricking the finger of a patient, placing the finger of the patient on the finger portion, collecting the blood, and de-assembling the blood collection device are all carried out by the patient him/herself.

Optionally, the finger of the user may be prepared before the step of pricking the finger. For example, the finger may be held under a running flow of hot water to enhance blood flow.

A fifth aspect of the present invention relates to a blood collection device for the self-collection of a blood sample by a user, the blood collection device comprising a base element, a collection container, and a finger portion,
- the base element including a hinge member for moving the base element between a folded state and an unfolded state (and vice versa) and an upstanding wall which defines a receive opening for receiving the collection container, the base element, in the unfolded state, having a width of more than 32 mm and, in the folded state, having a height of less than 32 mm;
- the collection container being configured for placement in the receive opening of the base element in a substantially upright orientation;
- the finger portion being configured for placement on top of the collection container and comprising a blood collection hole which, in an assembled state of the blood collection device, provides a fluid channel between the collection container and the finger portion,

wherein the base element is configured to be shipped to a user in the folded state, and
wherein the base element is configured to be unfolded by a user after receiving said blood collection device and before using said blood collection device.

The advantages described in the above in relation to the first aspect of the present invention are equally applicable to the second, third, fourth and fifth aspect of the present invention.

Specific embodiments described in relation to the first aspect are equally applicable to the second, third, fourth and fifth aspect of the present invention.

These and other aspects of the invention are elucidated further with respect to the below figures, wherein like parts are indicated with the same reference numerals. Herein,
Figures 1A and 1B schematically illustrate a side view of an embodiment of the blood collection device according to the present invention in both its assembled and its de-assembled state;
Figures 2A - 2D schematically illustrate isometric views of various embodiments of the blood collection device according to the present invention;
Figure 3 schematically illustrates a partial longitudinal cross-sectional view of an embodiment of the blood collection device according to the present invention;
Figure 4 schematically illustrates a top view of an assembly of an insert member and a capillary tube;
Figure 5 schematically illustrates an exploded view and an assembled state of an embodiment of a blood collection device according to the present invention;
Figure 6 schematically illustrates an embodiment of a blood collection kit according to the second aspect of the invention;
Figure 7 schematically illustrates a longitudinal cross-sectional view of an embodiment of the blood collection device according to the invention; and
Figure 8 schematically illustrates an embodiment of a blood collection device according to the invention, wherein a base element thereof is foldable.

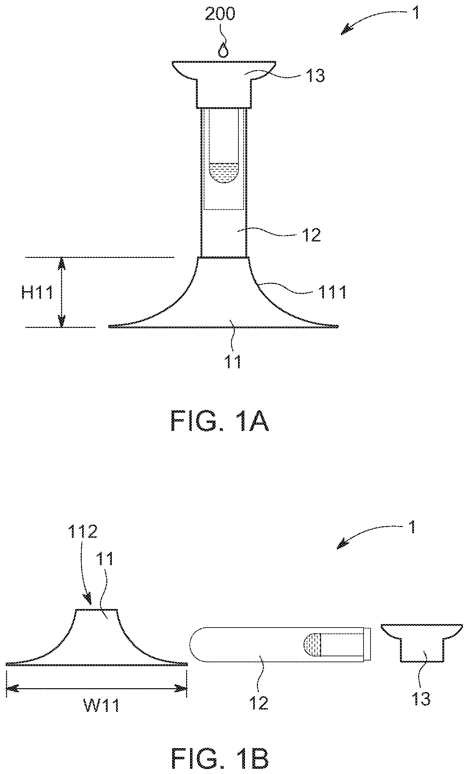

With reference to Figures 1A and 1B the blood collection device 1 generally comprises three components: a base element 11, a collection container 12 and a finger portion 13. Each of the different components will be described individually in more detail below. Importantly, at least the base element 11 is a separate part compared to the other components 12, 13. Also the collection container 12 and the finger portion 13 are here however separate parts compared to each other and compared to the base element 11. As the three components 11, 12, 13 are all separate parts, they can be assembled and de-assembled with respect to each other. This allows to ship the blood collection device 1 to be shipped to a user in a de-assembled state. As is visible in Figure 1B, in the de-assembled state the blood collection device 1 advantageously fits inside a box that has a height of 32 mm, a length of 380 mm (not indicated) and a length of 265 mm (not indicated). This allows the blood collection device to be send to a user via letterbox post (Dutch requirements for letterbox post at the date of filing the application; in other countries these dimensions may vary to a more or less degree. The inventive concept underlying the invention can of course easily be adapted to other national postal service requirements without departing from the scope of the invention). Due to the simplicity of the blood collection device 1, a user can very easily assemble the blood collection device 1 after having received it and before using it to collect a sample of blood 200.

The collection container 12 may be a standardized part and is preferably a "buy-in" piece. The advantage of using a standardized collection container is that it may be accepted for testing purposes by any blood testing laboratory. The finger portion 13 and the base element 11 may in turn e.g. be made via an injection process, or e.g. via 3D printing process.

As is indicated in Figures 1A and 1B, the base element 11 of the blood collection device 1 has a height H11. The height is less than 32 mm, preferably less than 28 mm e.g. less than 25 mm such that the base element 11 can be placed in e.g. a cardboard box and the resulting package is still thinner than 32 mm.

The height H11 of the base element 11 is defined by an upstanding wall 111, which is hollow at the inner side thereof and defines a receive opening 112 for receiving the collection container 12. Preferably the shape of the receive opening 112 is adapted to the shape of the collection container 12 such that the collection container 12 neatly fits inside the receive opening 112. Preferably the collection container 12 and the receive opening 112 are circular.

The base element 11 has a width W11 of more than 32 mm. This ensures a stable and trust-worthy design when the blood-collection device is assembled. For example, the width W11 of the base element 11 may be larger than 40mm, preferably the width W11 of the base element is larger than 50 mm, or even larger than 55 mm, such as about 58 mm.

Preferably the width W11 of the base element 11 is less than 250 mm, to allow it to fit within the above-mentioned postal dimensions for letterbox mail.

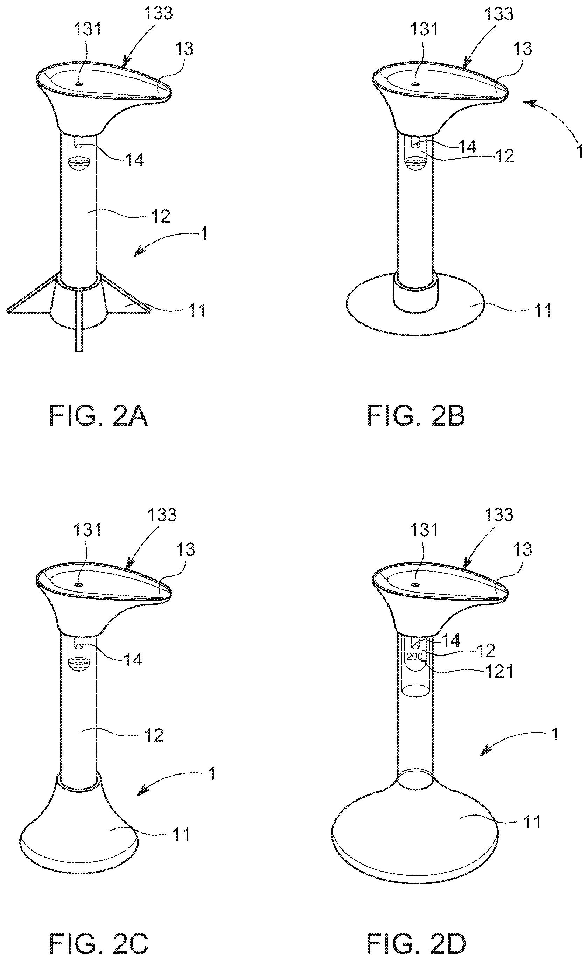

With reference to figures 2A - 2D, several different shapes of the base element 11 are schematically shown. In Figure 2A, the base element 11 is cross-shaped, having four legs (of which one is hidden from view). A width of the base element 11 is then defined from one outer end of a leg to the outer end of the opposing leg. Of course, the base element may also have any other number of legs, preferably at least three, such as three, four or five. A skilled person will find it very easy to determine the "width" of a cross-shaped base element 11 with an uneven amount of legs. It is in general at least equal to twice the net length of a leg, plus the diameter of the receive opening.

In figures 2B - 2D the shapes of the base elements 11 are comparable. They all have a circular base area, being sloped towards the receive opening in a more or less degree. In Figure 2B the upstanding wall extends from a base area at an angle of about 90°, whereas said angle is smaller in figure 2C and even smaller in figure 2D. The width of the base element 11 is smaller in figure 2C than in Figure 2D, with figure 2B in the middle. In terms of proportion, it is preferred when the width of the base element 11 is larger than a length of the finger portion 13.

Further visible in the isometric views of Figures 2A - 2D is the shape of the finger portion 13. The finger portion 13 comprises a blood collection hole 131 and can be placed on top of the collection container 12 in the assembled state of the blood collection device 1. An upper surface 133 of the finger portion 13 here has an elongated shape and is slanted, the lowest point of the upper surface 133 preferably being arranged at or near the blood collection hole 131.

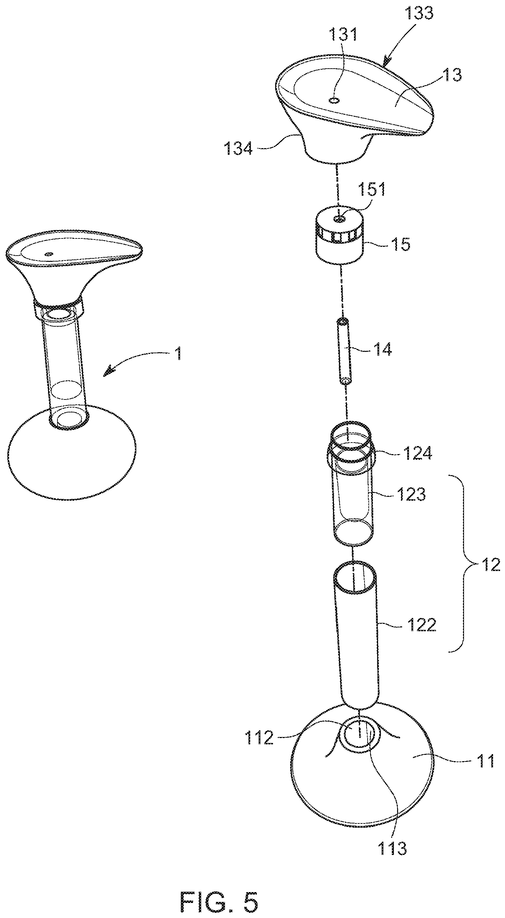

Further visible in the isometric views of Figures 2A - 2D is a transparent collection container 12. In the shown embodiments, said collection container may comprise an outer tube 122 (see e.g. also figure 5) which is placed in the base element 11 and an inner tube 123 (see e.g. also figure 5) which is used for the collection of the blood sample. The outer tube 122 may be a standardized part, which is accepted by any test laboratory for testing purposes. Also the inner tube 123 may be a standardized part. The outer tube (and thus the collection container 12) is placed in the receive opening of the base element 11. The outer tube and the inner tube are oriented in an upright position. Again with reference to the exploded view of Figure 5, the inner tube 123 may e.g. comprise an outer rim 124 that in an assembled state of the blood collection device rests on an upper face of the outer tube 122.

In figure 2D a bar line 121 is illustrated. The bar line 121 may e.g. be provided on the outer tube or on the inner tube and indicates a fill level of the collection container 12. In the shown embodiment the "200" may e.g. indicate that when the collection container 12 is filled until bar line 121, 200 µl of blood is collected.

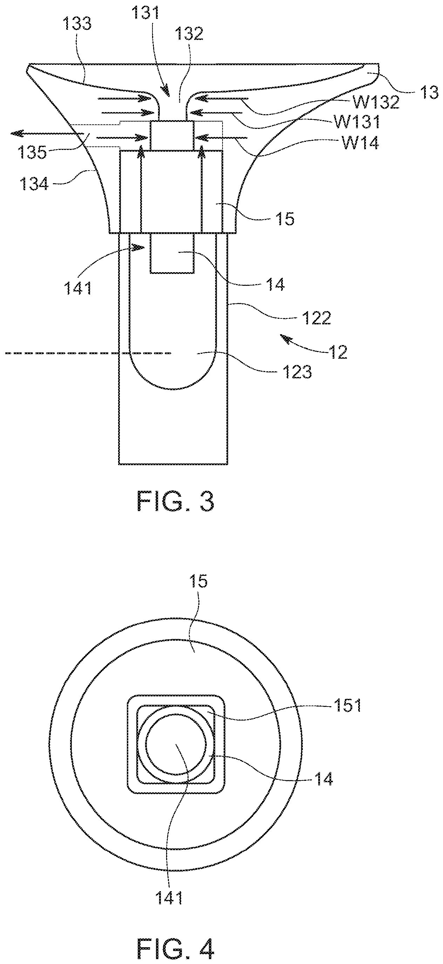

Further visible in Figures 2A - 2D is the capillary tube 14, which will be discussed in more detail with respect to figures 3 and 4. The capillary tube 14 is preferably smaller in diameter than the collection container 12 and preferably extends into the collection container 12, as is shown here.

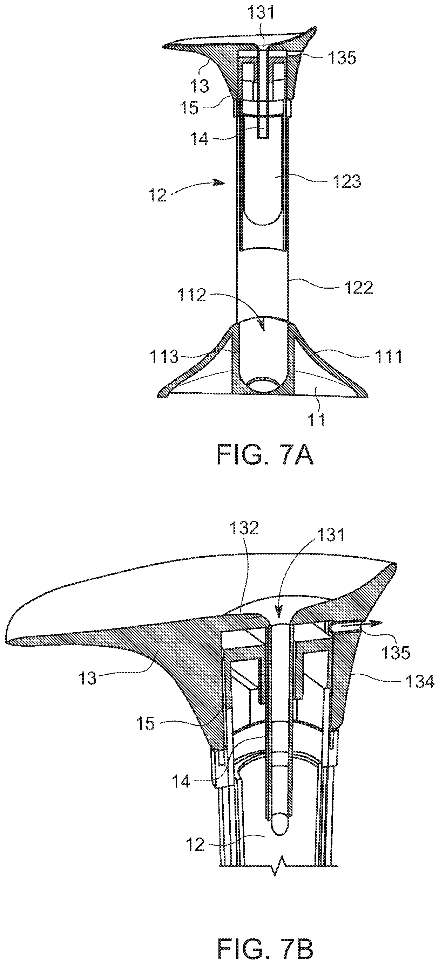

Turning to figures 3, 7A and 7B now, which will be described simultaneously, a longitudinal cross-sectional view of the blood collection device 1 is visible. Figures 3 and 7B shows the finger portion 13, the capillary tube 14, the insert member 15, and the collection container 12 with outer tube 122 and inner tube 123 in an assembled state. Figure 7A shows the entire blood collection device 1. As visible, the upper surface 133 of the finger portion 13 is slanted and directed towards blood collection hole 131. In the shown embodiments the finger portion 13 comprises a blood collection channel 132, formed by a downwardly extending wall and the blood collection hole 131 at the bottom of it. The shape of the blood collection channel 132 is here conical, a width W132 of the channel 132 decreasing in a direction from the upper surface 133 of the finger portion 13 towards the collection container 12.

In the present embodiment, the width of the blood collection channel 132 at the blood collection hole 131, denoted W131 and here the smallest width of the blood collection channel 132, is larger than an outer width W14 of the capillary tube 14. For example, the width W131 of the blood collection hole 131 may be about 3 mm. This difference in size between the capillary tube 14 and the blood collection hole 131 ensures that the capillary tube 14 advantageously abuts the blood collection channel 132 and does not extend up to or beyond the upper surface 133 of the finger portion 13.

Further visible in Figures 3, 7A and 7B is an air outlet hole 135. Said air outlet hole 135 may assist any air in the collection container 12 and/or in the inner tube 122 of the collection container 12 to leave said collection container 12 and/or inner tube 122 such that blood may more easily flow from the finger portion 13 into the collection container 12. In other words, any overpressure in the collection container 12, created by excess air therein, may be resolved by having an air outlet 135 in the finger portion 13. The flow of excess air is schematically illustrated by arrows in Figure 3. The air outlet hole 135 may e.g. have a diameter of between 0.5 mm and 3 mm, such as approximately 1 mm or approximately 2 mm.

As shown, the capillary tube 14 provides a fluid channel 141 for blood between the finger portion 13 and the collection container 12.

As shown, the bottom side of the finger portion 13 is hollow, such that the insert member 15 may be inserted therein.

As is shown in Figure 7A, the base element 11 may comprise a receive pocket 113 including the receive opening 112 for receiving the collection container 12 in the assembled state of the blood collection device 1.

It is preferred that the finger portion 13, the insert member 15 and the capillary tube 14 are pre-assembled before being shipped to the user.

As is better visible in Figure 4, said insert member 15 comprises a capillary tube receiving channel 151 for receiving and fixating the capillary tube 14 therein. Via the insert member 15, the capillary tube 14 may thus be fixed with respect to the finger portion 13.

Whereas the cross-section of the capillary tube receiving channel 151 of the insert member 15 is substantially square, the cross section of the capillary tube 15 is substantially circular. As the width of the channel 151 substantially matches the outer diameter of the capillary tube 14, said capillary tube 14 may be press-fit in the capillary tube receiving channel 151.

Figure 5 again shows a schematic overview of the blood collection device 1, wherein figure 5A shows the blood collection device 1 in an assembled state, and figure 5B shows an exploded view thereof.

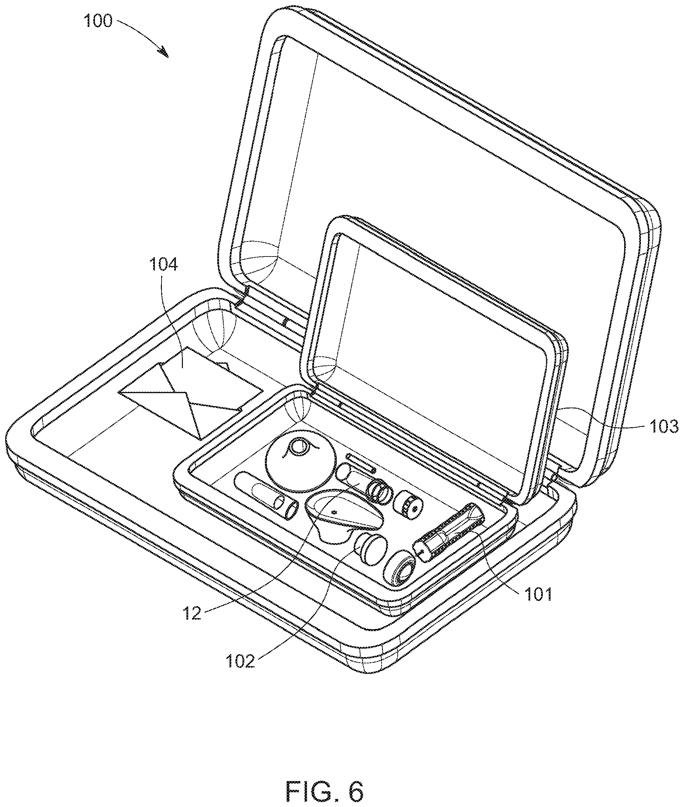

Figure 6 shows a blood collection kit 100 which comprises the blood collection device 1 as described in the above, a lancet 101, a cap 102 for sealing the collection container 12 after blood is collected therein, and a return envelope 104. Optionally also an analysis tool 105 (not shown) for self-analysis of the collected blood may be part of the blood collection kit 100. Preferably at least the lancet 101, the cap 102 and the components of the blood collection device 1 are packed in a sterilized bag 103.

A blood sample may be collected by a user by:
- firstly shipping a blood collection device 1 or a blood collection kit 100 to a user by letterbox post, the blood collection device 1 being in a de-assembled state during shipment;
- secondly assembling the blood collection device 1, preferably by the user;
- thirdly pricking a finger (schematically illustrated by the drop of blood 200 in Figure 1) of a patient to initiate a blood flow;, preferably by the user;
- fourthly placing the finger 200 of the patient on the finger portion 13 of the blood collection device 1, preferably by the user;
- fifthly collecting the blood of the patient in the collection container 12, preferably by the user;
- sixthly de-assembling the blood collection device 1, preferably by the user;
- seventhly sealing the collection container 12 with a cap 102, preferably by the user; and
- finally shipping the collection container 12, inclusive the blood collected therein, to a blood analysis laboratory where the testing can be performed, preferably by the user. In an embodiment the collection container 12, comprising outer tube 122 and inner tube 123 may be send back in its entirety. In another embodiment, only the inner tube 123 may be returned by the user.

Optionally the blood collection kit 100 comprises a self-testing tool, which can be used by the user to perform a test on the collected blood.

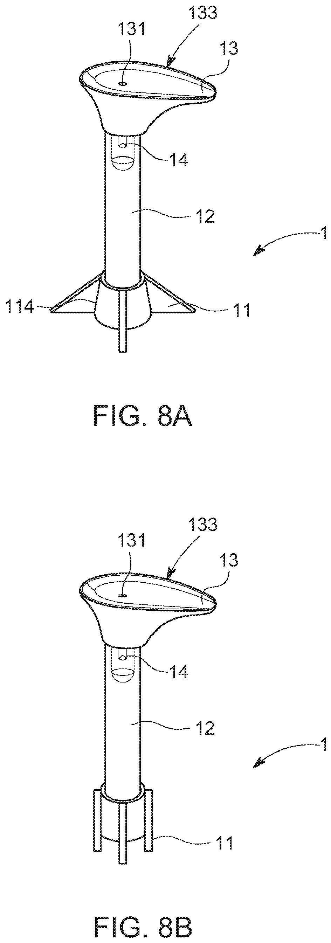

Figure 8A finally shows an embodiment of a blood collection device 1 which is similar to the embodiment of figure 2A. The base element 11 of the blood collection device 1 of figure 8A however additionally comprises a hinge member 114 which allows the base element 11 to be moved between a folded state and an unfolded state (and vice versa), such that the base element 11 may be shipped to a user in a compressed, i.e. folded, state and be unfolded by a user after receiving the blood collection device 1.

It is noted that in the shown embodiment the base element 11 may also be de-assembled with respect to the collection container 12, but this is not required when the base element 11 may be compressed by folding in hinge members 114.

### LIST OF REFERENCE NUMERALS

- 1: Blood collection device
11 base element
W11 width of base element
H11 height of base element
111 upstanding wall
112 receive opening
113 receive pocket
114 hinge member
12 collection container
121 barline
122 outer tube
123 inner tube
124 outer rim of inner tube
13 finger portion
131 blood collection hole
W131 width blood collection hole
132 blood collection channel
W132 width blood collection channel
133 upper surface
134 side wall
135 air outlet hole
14 capillary tube
141 fluid channel
15 insert member
151 capillary tube receiving channel

- 100: blood collection kit
101 lancet
102 cap
103 sterilized bag
104 return envelope

- 200: finger

## Claims

1. A blood collection device (1) for the self-collection of a blood sample by a user, the blood collection device comprising a base element (11), a collection container (12), and a finger portion (13),
- the base element (11) having a width (W11) of more than 32 mm and including an upstanding wall (111) which defines a receive opening (112) for receiving the collection container (12), the upstanding wall (111) having a height (H11) of less than 32 mm;
- the collection container (12) being configured for placement in the receive opening (112) of the base element (11) in a substantially upright orientation;
- the finger portion (13) being configured for placement on top of the collection container (12) and comprising a blood collection channel (132) including a blood collection hole (131), wherein the blood collection hole (131), in an assembled state of the blood collection device (1), enables a flow of blood from the finger portion (13) into the collection container (12);
wherein at least the base element (11) is a separate part, configured to be shipped to a user in a de-assembled state with respect to the other parts (12, 13) of the blood collection device (1), and
wherein the blood collection device (1) is configured to be assembled by a user after receiving said blood collection device (1) and before using said blood collection device (1),
**characterised, in that** a width (W132) of the blood collection channel (132) decreases in a direction from an upper surface (133) of the finger portion (13) to the collection container (12).

2. The blood collection device according to claim 1, further comprising a capillary tube (14) that, in an assembled state, is arranged between the blood collection hole (131) and the collection container (12), the capillary tube (14) defining a fluid channel (141).

3. The blood collection device according claim 2, wherein an outer width (W14) of the capillary tube (14) is larger than a width (W131) of the blood collection hole (131).

4. The blood collection device according to any of the preceding claims, wherein a side wall (134) of the finger portion (13) comprises an air outlet hole (135).

5. The blood collection device according to any of the claims 2-4, further comprising an insert member (15) configured to be inserted into the finger portion (13) and for receiving the capillary tube (14), to fixate the capillary tube (14) with respect to the finger portion (13).

6. The blood collection device according to claim 5, wherein a capillary tube receiving channel (151) of the insert member (15) has a non-circular cross-section and wherein the capillary tube (14) has a circular cross-section.

7. The blood collection device according to claim 5 or 6, wherein the finger portion (13), the insert member (15) and the capillary tube (14) are pre-assembled in the de-assembled state.

8. The blood collection device according to any of the preceding claims, wherein an upper surface (133) of the finger portion (13) has an elongate shape

9. The blood collection device according to any of the preceding claims, wherein a width (W11) of the base element (11) is larger than 40 mm, preferably larger than 50 mm, such as larger than 55 mm.

10. The blood collection device according to any of the preceding claims, wherein the base element (11) comprises a receive pocket (113) including the receive opening (112), for receiving the collection container (12) in the assembled state of the blood collection device (1).

11. The blood collection device according to any of the preceding claims, wherein the base element (11) and/or the finger portion (13) are manufactured via an injection moulding process, the base element (11) and the finger portion (13) preferable being made from polypropylene.

12. The blood collection device according to any of the preceding claims, wherein the base element (11) and/or the finger portion (13) are manufactured via an 3D printing process, preferably via a selective laser sintering process, the base element (11) and the finger portion (13) preferable being made from PA2200.

13. Blood collection kit (100) comprising the blood collection device (1) according to any of the claims 1 - 12, a lancet (101), and a cap (102) for sealing the collection container (12), preferably packed in a sterilized bag (103), the blood collection kit (100) preferably also comprising a return envelope (104) and/or an analysis tool (105) for self-analysis of the collected blood sample.

14. Method for collecting a blood sample, comprising the steps of:
- shipping a blood collection device (1) according to any one of the claims 1 - 12 or a blood collection kit (100) according to claim 13 to a user by letterbox post, a base element (11) of the blood collection device (1) being de-assembled with respect to the other components of the blood collection device (1) during shipment,
- assembling the blood collection device (1);
- pricking a finger (200) of a patient to initiate a blood flow;
- placing the finger (200) of the patient on the finger portion (13) of the blood collection device (1);
- collecting the blood of the patient in the collection container (12);
- de-assembling the blood collection device (1);
- returning the collection container (12), inclusive of the blood collected therein;
wherein the steps of assembling the blood collection device (1), pricking the finger (200) of a patient, placing the finger (200) of the patient on the finger portion (13), collecting the blood, and de-assembling the blood collection device (1) are all carried out by the patient him/herself.

## Patentansprüche

1. Blutentnahmevorrichtung (1), damit ein Benutzer sich selbst eine Blutprobe abnehmen kann, wobei die Blutentnahmevorrichtung ein Basiselement (11), einen Sammelbehälter (12) und einen Fingerabschnitt (13) umfasst,
- wobei das Basiselement (11) eine Breite (W11) von mehr als 32 mm hat und eine aufrechte Wand (111) aufweist, die eine Aufnahmeöffnung (112) zur Aufnahme des Sammelbehälters (12) definiert, wobei die aufrechte Wand (111) eine Höhe (H11) von weniger als 32 mm hat,
- wobei der Sammelbehälter (12) zur Platzierung in der Aufnahmeöffnung (112) des Basiselements (11) in einer im Wesentlichen aufrechten Ausrichtung ausgestaltet ist,
- der Fingerabschnitt (13) zur Platzierung auf dem Sammelbehälter (12) ausgestaltet ist und einen Blutentnahmekanal (132) umfasst, der ein Blutentnahmeloch (131) aufweist, wobei das Blutentnahmeloch (131) in einem zusammengebauten Zustand der Blutentnahmevorrichtung (1) ein Strömen von Blut von dem Fingerabschnitt (13) in den Sammelbehälter (12) ermöglicht,
wobei mindestens das Basiselement (11) ein separates Teil ist, das dazu ausgestaltet ist, an einen Benutzer in einem auseinandergebauten Zustand bezüglich der anderen Teile (12, 13) der Blutentnahmevorrichtung (1) verschickt zu werden, und
wobei die Blutentnahmevorrichtung (1) dazu ausgestaltet ist, von einem Benutzer nach dem Empfang der Blutentnahmevorrichtung (1) und vor der Verwendung der Blutentnahmevorrichtung (1) zusammengebaut zu werden,
**dadurch gekennzeichnet, dass** eine Breite (W132) des Blutentnahmekanals (132) in einer Richtung von einer oberen Fläche (133) des Fingerabschnitts (13) zu dem Sammelbehälter (12) abnimmt.

2. Blutentnahmevorrichtung nach Anspruch 1, ferner umfassend ein Kapillarrohr (14), das in einem zusammengebauten Zustand zwischen dem Blutentnahmeloch (131) und dem Sammelbehälter (12) angeordnet ist, wobei das Kapillarrohr (14) einen Fluidkanal (141) definiert.

3. Blutentnahmevorrichtung nach Anspruch 2, wobei eine äußere Breite (W14) des Kapillarrohrs (14) größer als eine Breite (W131) des Blutentnahmelochs (131) ist.

4. Blutentnahmevorrichtung nach einem der vorhergehenden Ansprüche, wobei eine Seitenwand (134) des Fingerabschnitts (13) ein Luftauslassloch (135) umfasst.

5. Blutentnahmevorrichtung nach einem der Ansprüche 2 - 4, ferner umfassend ein Einführglied (15), das dazu ausgestaltet ist, in den Fingerabschnitt (13) eingeführt zu werden und das Kapillarrohr (14) aufzunehmen, um das Kapillarrohr (14) bezüglich des Fingerabschnitts (13) festzulegen.

6. Blutentnahmevorrichtung nach Anspruch 5, wobei ein Kapillarrohraufnahmekanal (151) des Einführglieds (15) einen nicht kreisförmigen Querschnitt hat und wobei das Kapillarrohr (14) einen kreisförmigen Querschnitt hat.

7. Blutentnahmevorrichtung nach Anspruch 5 oder 6, wobei der Fingerabschnitt (13), das Einführglied (15) und das Kapillarrohr (14) in dem auseinandergebauten Zustand vormontiert sind.

8. Blutentnahmevorrichtung nach einem der vorhergehenden Ansprüche, wobei eine obere Fläche (133) des Fingerabschnitts (13) eine längliche Form hat.

9. Blutentnahmevorrichtung nach einem der vorhergehenden Ansprüche, wobei eine Breite (W11) des Basiselements (11) größer als 40 mm, vorzugsweise größer als 50 mm, wie größer als 55 mm, ist.

10. Blutentnahmevorrichtung nach einem der vorhergehenden Ansprüche, wobei das Basiselement (11) eine die Aufnahmeöffnung (112) aufweisende Aufnahmetasche (113) zur Aufnahme des Sammelbehälters (12) in dem zusammengebauten Zustand der Blutentnahmevorrichtung (1) umfasst.

11. Blutentnahmevorrichtung nach einem der vorhergehenden Ansprüche, wobei das Basiselement (11) und/oder der Fingerabschnitt (13) über ein Spritzgussverfahren hergestellt werden, wobei das Basiselement (11) und der Fingerabschnitt (13) vorzugsweise aus Polypropylen hergestellt sind.

12. Blutentnahmevorrichtung nach einem der vorhergehenden Ansprüche, wobei das Basiselement (11) und/oder der Fingerabschnitt (13) über ein 3D-Druckverfahren, vorzugsweise über ein selektives Lasersinterverfahren, hergestellt werden, wobei das Basiselement (11) und der Fingerabschnitt (13) vorzugsweise aus PA2200 hergestellt sind.

13. Blutentnahmesatz (100), umfassend die Blutentnahmevorrichtung (1) nach einem der Ansprüche 1 - 12, eine Lanzette (101) und eine Kappe (102) zum Abdichten des Sammelbehälters (12), der vorzugsweise in einem sterilisierten Beutel (103) verpackt ist, wobei der Blutentnahmesatz (100) vorzugsweise auch einen Rückumschlag (104) und/oder ein Analysewerkzeug (105) zur Selbstanalyse der entnommenen Blutprobe umfasst.

14. Verfahren zur Entnahme einer Blutprobe, umfassend die folgenden Schritte:
- Versenden einer Blutentnahmevorrichtung (1) nach einem der Ansprüche 1 - 12 oder eines Blutentnahmesatzes (100) nach Anspruch 13 an einen Benutzer per Briefkastenpost, wobei ein Basiselement (11) der Blutentnahmevorrichtung (1) während des Versands nicht mit den anderen Komponenten der Blutentnahmevorrichtung (1) zusammengebaut ist,
- Zusammenbauen der Blutentnahmevorrichtung (1),
- Einstechen in den Finger (200) eines Patienten, um das Strömen von Blut einzuleiten,
- Platzieren des Fingers (200) des Patienten auf den Fingerabschnitt (13) der Blutentnahmevorrichtung (1),
- Sammeln des Bluts des Patienten in dem Sammelbehälter (12),
- Auseinanderbauen der Blutentnahmevorrichtung (1),
- Rücksenden des Sammelbehälters (12) einschließlich des darin gesammelten Bluts,
wobei die Schritte des Zusammenbauens der Blutentnahmevorrichtung (1), des Einstechens in den Finger (200) eines Patienten, des Platzierens des Fingers (200) des Patienten auf den Fingerabschnitt (13), des Sammelns des Bluts und des Auseinanderbauens der Blutentnahmevorrichtung (1) alle von dem Patienten selbst durchgeführt werden.

## Revendications

1. Dispositif de collecte de sang (1) pour l'auto-collecte d'un échantillon de sang par un utilisateur, le dispositif de collecte de sang comprenant un élément de base (11), un récipient de collecte (12) et une partie doigt (13),
- l'élément de base (11) ayant une largeur (W11) supérieure à 32 mm et comprenant une paroi verticale (111) qui définit une ouverture de réception (112) pour recevoir le récipient de collecte (12), la paroi verticale (111) ayant une hauteur (H11) inférieure à 32 mm ;
- le récipient de collecte (12) étant configuré pour être placé dans l'ouverture de réception (112) de l'élément de base (11) dans une orientation sensiblement verticale ;
- la partie doigt (13) étant configurée pour être placée sur la partie supérieure du récipient de collecte (12) et comprenant un canal de collecte de sang (132) comprenant un trou de collecte de sang (131), où le trou de collecte de sang (131), dans un état assemblé du dispositif de collecte de sang (1), permet un écoulement de sang depuis la partie doigt (13) dans le récipient de collecte (12) ;
dans lequel au moins l'élément de base (11) est une partie séparée, configurée pour être expédiée à un utilisateur dans un état désassemblé par rapport aux autres parties (12, 13) du dispositif de collecte de sang (1), et
dans lequel le dispositif de collecte de sang (1) est configuré pour être assemblé par un utilisateur après avoir reçu ledit dispositif de collecte de sang (1) et avant d'utiliser ledit dispositif de collecte de sang (1),
**caractérisé en ce que,** une largeur (W132) du canal de collecte de sang (132) diminue dans une direction allant d'une surface supérieure (133) de la partie doigt (13) vers le récipient de collecte (12).

2. Dispositif de collecte de sang selon la revendication 1, comprenant en outre un tube capillaire (14) qui, dans un état assemblé, est agencé entre le trou de collecte de sang (131) et le récipient de collecte (12), le tube capillaire (14) définissant un canal de fluide (141).

3. Dispositif de collecte de sang selon la revendication 2, dans lequel une largeur externe (W14) du tube capillaire (14) est supérieure à une largeur (W131) du trou de collecte de sang (131).

4. Dispositif de collecte de sang selon l'une des revendications précédentes, dans lequel une paroi latérale (134) de la partie doigt (13) comprend un trou de sortie d'air (135).

5. Dispositif de collecte de sang selon l'une des revendications 2 à 4, comprenant en outre un élément d'insertion (15) configuré pour être inséré dans la partie doigt (13) et pour recevoir le tube capillaire (14), afin de fixer le tube capillaire (14) par rapport à la partie doigt (13).

6. Dispositif de collecte de sang selon la revendication 5, dans lequel un canal de réception de tube capillaire (151) de l'élément d'insertion (15) a une section transversale non circulaire et dans lequel le tube capillaire (14) a une section transversale circulaire.

7. Dispositif de collecte de sang selon la revendication 5 ou 6, dans lequel la partie doigt (13), l'élément d'insertion (15) et le tube capillaire (14) sont pré-assemblés à l'état désassemblé.

8. Dispositif de collecte de sang selon l'une des revendications précédentes, dans lequel une surface supérieure (133) de la partie doigt (13) a une forme allongée.

9. Dispositif de collecte de sang selon l'une des revendications précédentes, dans lequel une largeur (W11) de l'élément de base (11) est supérieure à 40 mm, de préférence supérieure à 50 mm, par exemple supérieure à 55 mm.

10. Dispositif de collecte de sang selon l'une des revendications précédentes, dans lequel l'élément de base (11) comprend une poche de réception (113) comprenant l'ouverture de réception (112), pour recevoir le récipient de collecte (12) dans l'état assemblé du dispositif de collecte de sang (1).

11. Dispositif de collecte de sang selon l'une des revendications précédentes, dans lequel l'élément de base (11) et/ou la partie doigt (13) sont fabriqués par un processus de moulage par injection, l'élément de base (11) et la partie doigt (13) étant de préférence réalisés en polypropylène.

12. Dispositif de collecte de sang selon l'une des revendications précédentes, dans lequel l'élément de base (11) et/ou la partie doigt (13) sont fabriqués via un processus d'impression 3D, de préférence via un processus de frittage laser sélectif, l'élément de base (11) et la partie doigt (13) étant de préférence réalisés en PA2200.

13. Kit de collecte de sang (100) comprenant le dispositif de collecte de sang (1) selon l'une des revendications 1 à 12, une lancette (101) et un capuchon (102) pour fermer hermétiquement le récipient de collecte (12), de préférence emballé dans une poche stérilisée (103), le kit de collecte de sang (100) comprenant de préférence également une enveloppe de retour (104) et/ou un outil d'analyse (105) pour l'auto-analyse de l'échantillon de sang collecté.

14. Procédé de collecte d'un échantillon de sang, comprenant les étapes de :
- expédition d'un dispositif de collecte de sang (1) selon l'une quelconque des revendications 1 à 12 ou d'un kit de collecte de sang (100) selon la revendication 13 à un utilisateur par courrier postal, un élément de base (11) du dispositif de collecte de sang (1) étant désassemblé par rapport aux autres composants du dispositif de collecte de sang (1) pendant l'expédition,
- assemblage du dispositif de collecte de sang (1) ;
- piqûre d'un doigt (200) d'un patient pour déclencher un écoulement de sang ;
- placement du doigt (200) du patient sur la partie doigt (13) du dispositif de collecte de sang (1) ;
- collecte du sang du patient dans le récipient de collecte (12) ;
- désassemblage du dispositif de collecte de sang (1) ;
- retour du récipient de collecte (12), y compris le sang qui y a été collecté ;
dans lequel les étapes d'assemblage du dispositif de collecte de sang (1), de piqûre du doigt (200) d'un patient, de placement du doigt (200) du patient sur la partie doigt (13), de collecte du sang et de désassemblage du dispositif de collecte de sang (1) sont toutes réalisées par le patient lui-même.
